# EUROPEAN PATENT APPLICATION

(11) **EP 3 970 667 A1**
(43) Date of publication of application: **23.03.2022**
(21) Application number: 21785741.6
(22) Date of filing: 18.01.2021
(51) Int. Cl.: A61F 2/24

(54) **VALVE STENT AND ARTIFICIAL HEART VALVE CONTAINING SAME**

(30) Priority: 21.07.2020 CN 202021443542 U; 21.07.2020 CN 202010704557
(71) Applicant: Jiangsu Trulive Medtech Co., Ltd, Rudong County Nantong Jiangsu 226400 (CN)
(72) Inventor: ZHAO, Jing, Jiangsu 226400 (CN)
(74) Representative: Argyma
(86) International application number: PCT/CN2021/072492
(87) International publication number: WO 2022/016837

(57) **Abstract**

A valve stent includes a stent assembly. The stent assembly includes at least two stents: an inner layer stent and an outer layer stent, and the inner layer stent and the outer layer stent are of an internal-external nested structure. The stent assembly is configured to have at least one sheath inserting end set including a first sheath inserting end and a second sheath inserting end, and the second sheath inserting end is located on an outer side of the first sheath inserting end. In at least one sheath inserting end set, a length of the first sheath inserting end is greater than a length of the second sheath inserting end, and the sheath inserting end set is configured with a projecting portion for preventing the loading of a valve prosthesis from being interfered by the sheath inserting end set.

## Description

### BACKGROUND OF THE INVENTION

### Field of the Invention

This invention relates to the technical field of medical instruments, and more specifically, to a valve stent and a prosthetic heart valve implanted into a heart.

### Description of the Prior Art

A heart valve is a membrane-like structure that can be opened and closed inside the organs of a person or certain animals. Each person has four valves in his or her heart, which are an aortic valve linking a left ventricle to an aorta, a pulmonary valve linking a right ventricle to a pulmonary artery, a mitral valve linking a left atrium to the left ventricle, and a tricuspid valve linking a right atrium to the right ventricle. They all act as one-way valves, allowing blood to flow from one direction to another without reflux.

The mitral regurgitation may cause myocardial remodeling, lead the ventricle to dilate progressively, and finally cause heart failure. The transcatheter mitral valve replacement (TMVR) adopts a method of intervening a catheter, wherein the prosthetic valve is compressed to a delivery system in vitro and transported to the human mitral valve annulus, and then the prosthetic valve is released and fixed at the mitral valve annulus to replace the native valve. Compared with surgery, TMVR does not require extracorporeal circulation devices, has a small trauma, a quick recovery for the patients, and can significantly improve the hemodynamic index of the patients after operation.

For some patients with mitral regurgitation, the treatment effect of implanting the traditional single-layered mitral prosthesis valve is not ideal. For the multi-layered mitral prosthesis valve, the functions of carrying the prosthetic valve leaflet, anchoring and sealing may be assigned to different single-layered valve components, so as to achieve the purpose of not affecting the normal operation of other structures of the heart and better exerting the function of implantation treatment.

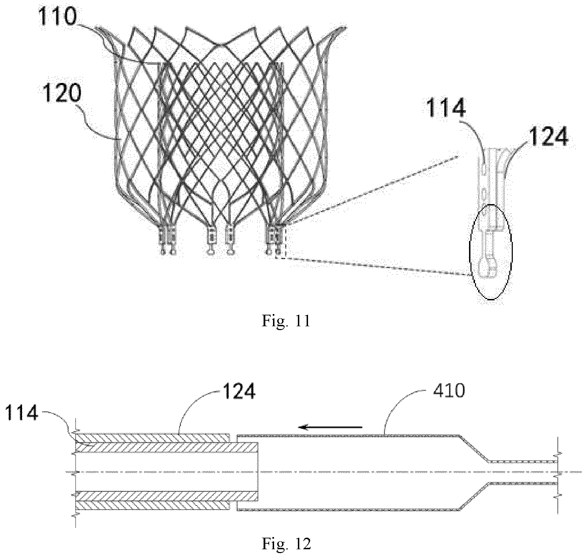

Loading the multi-layered stent is often difficult; with reference to Figs. 11 and 12, a double-layered stent structure is shown, wherein when a length of a sheath inserting end of an inner layer stent is greater than that of an outer layer stent, a gradient of sheath inserting end surfaces may be formed between the inner and outer stents. When being loaded, an end surface of the outer layer stent may touch an end surface of a sheath, and the two end surfaces may abut against each other, so that the entire valve may be affected in entering the sheath, the loading resistance is large and the outer layer stent may easily damage the sheath while causing a risk where the sheath enters an interlayer of two layer stents. During the implanting process, a retrievable resistance of the end portion is large if the retrieval and deployment are required, which will also affect the smooth progress of the retrieval.

### SUMMARY OF THE INVENTION

This invention provides a valve stent, which eliminates the gradient of the sheath inserting end surface between the two layer stents and avoids a condition that the end of the outer layer stent touches an end of the sheath to affect entering the stent into the sheath, thereby reducing the loading resistance and the risk of the sheath entering an interlayer.

The technical solution of this invention is as follows:
A valve stent includes a stent assembly, wherein the stent assembly includes at least two stents: an inner layer stent and an outer layer stent, and the inner layer stent and the outer layer stent are of an internal-external nested structure; the inner layer stent is sleeved inside, and the stent assembly is configured to have at least one sheath inserting end set;
the sheath inserting end set comprises a first sheath inserting end and a second sheath inserting end, the first sheath inserting end is located at the inner layer stent, and the second sheath inserting end is located at the outer layer stent; the first sheath inserting end and the second sheath inserting end extend in a same direction, and the second sheath inserting end is located on an outer side of the first sheath inserting end; in the at least one sheath inserting end set, when a length of the first sheath inserting end is greater than a length of the second sheath inserting end, the sheath inserting end set is configured with a projecting portion for preventing the loading of a valve prosthesis from being interfered by the sheath inserting end set.

Since there is a gradient difference between the first sheath inserting end and the second sheath inserting end, when the valve prosthesis is pressed and held into the sheath, the end surface of the second sheath inserting end may touch a delivery system such as the end surface of the sheath, and the two end surfaces abut against each other to enlarge the loading resistance, so that the entire valve prosthesis is affected from entering into the sheath and the second sheath inserting end may easily damage the sheath while resulting into the risk of the sheath entering an interlayer of the inner and outer stents. The projecting portion removes the interference of the sheath inserting end set in loading the valve prosthesis, and reduces the risk of the sheath entering an interlayer of the inner and outer stents, and of the end surface of the second sheath inserting end abutting against the sheath during the loading process.

One end of the valve prosthesis first enters the delivery system, and the end entering the delivery system first is the sheath inserting end. The first sheath inserting end may be a hanging tab disposed at the inner layer stent, and the stent assembly is connected to the delivery system by the hanging tab. Specific structures and functions of the first sheath inserting end and the second sheath inserting end may not be used to limit the protection scope of this invention.

According to the structure of the delivery system and different releasing ways, the valve prosthesis may be such that one end enters the sheath first; for example, the ventricle end enters the sheath first, or the atrium end enters the sheath first. When the delivery system such as the sheath is a two-segment structure, the ventricle end and the atrium end of the stent assembly, as the sheath inserting ends simultaneously, are loaded into the delivery system.

Preferably, the stent assembly includes a plurality of the sheath inserting end sets, and each of the sheath inserting end sets is located at an atrium end or a ventricle end, wherein the plurality of the sheath inserting end sets may ensure that the valve prosthesis is stably loaded into the delivery system, and the plurality of the sheath inserting end sets may be located at the atrium end simultaneously or at the ventricle end simultaneously or are arranged at the atrium end and the ventricle end respectively; in each of the sheath inserting end sets, when the length of the first sheath inserting end is greater than the length of the second sheath inserting end, each of the sheath inserting end sets is configured with the projecting portion. Each projecting portion may remove the interference of each of the sheath inserting end sets on the processing of entering the sheath.

Preferably, the projecting portion is arranged at the first sheath inserting end, the projecting portion is provided with a first end surface, the first end surface is located on a side close to an end surface of the second sheath inserting end, and a width of the first end surface is not smaller than a width of the second sheath inserting end. Then, since the projecting portion functions as a stopper, the sheath may not enter the interlayer from between the first sheath inserting end and the second sheath inserting end, thereby reducing the risk of the sheath entering an interlayer of the inner and outer stents.

Preferably, the projecting portion is further provided with a second end surface, the second end surface is located on a side deviated from the first end surface, and the second end surface is flush with the end surface of the first sheath inserting end. Then, the projecting portion removes the gradient difference between the first sheath inserting end and the second sheath inserting end, which further reduces the risk of the sheath entering an interlayer of the inner and outer stents, and when a length of the first end surface of the projection portion is greater than or equal to the width of the second sheath inserting end, the end surface of the second sheath inserting end may not touch the end surface of the sheath, thereby avoiding the end surface of the second sheath inserting end from abutting against the end surface of the sheath and reducing the loading resistance while avoiding the second sheath inserting end from damaging the sheath.

Preferably, the first end surface of the projecting portion abuts against the end surface of the second sheath inserting end. Since being subjected to the abutting action from the projecting portion, when entering the sheath, the sheath may not enter the interlayer of the stents from the end of the second sheath inserting end.

Preferably, the first end surface is matched with the end surface of the second sheath inserting end. Matching refers to the same size and to that the shape of the end surface forms a concave-convex match. In this way, when the projecting portion abuts against the end surface of the second sheath inserting end, the sheath inserting end set may form an integral sheath inserting, so that loading the valve prosthesis is easier while reducing the risk of the second sheath inserting end damaging the sheath.

Preferably, an outer contour of the projecting portion is an arc shape. The outer contour of the arc shape reduces the friction with the end of the sheath and the sheath wall, and reduces the loading resistance.

Preferably, the projecting portion is formed integrally at the first sheath inserting end, so that the molding process is simple, and the connection between the projection portion and the first sheath inserting end is more firm and reliable.

Preferably, the first sheath inserting end is a hanging tab connected to a delivery system. When the length of the first sheath inserting end of the inner layer stent is longer, the first sheath inserting end is connected to the delivery system preferably by the hanging tab of the inner layer stent, which is more reliable and stable.

Preferably, an axial contact surface of the first sheath inserting end and the second sheath inserting end is configured to be a concave-convex matched connection. When the valve stent is molded, in each of the sheath inserting end sets, the first sheath inserting end and the second sheath inserting end may be pre-finalized by way of welding, and the concave-convex matched connection may replace the connection way of welding while having advantages of stable connection and being easy to implement.

A prosthetic heart valve includes the valve stent according to any one of the above descriptions.

Compared with the conventional art, this invention has the following beneficial effects:
First, in this invention, the projecting portion removes the interference of the sheath inserting end set in loading the valve prosthesis, and reduces the risk of the sheath entering an interlayer of the inner and outer stents, and of the end surface of the second sheath inserting end abutting against the sheath during the loading process; the projection portion is disposed at the first sheath inserting end, and the projecting portion may effectively prevent the sheath from entering the interlayer of the stents when the length of the first end surface is not smaller than the width of the second sheath inserting end; when the second end surface of the projecting portion is flush with the end surface of the first sheath inserting end, the projecting portion removes the gradient difference between the first sheath inserting end and the second sheath inserting end, then the end surface of the second sheath inserting end may not touch the end surface of the sheath, thereby avoiding the end surface of the second sheath inserting end from abutting against the end surface of the sheath and reducing the loading resistance while reducing the risk of damaging the sheath.

Second, when the first end surface of the projecting portion abuts against the end surface of the second sheath inserting end and the first end surface is matched with the end surface of the second sheath inserting end, an integrity of the sheath inserting end set is improved, and the risk of the second sheath inserting end damaging the sheath is further reduced, thereby ensuring that the valve prosthesis may be smoothly loaded, transported and deployed, and the sheath may be smoothly retrieved; when the outer contour of the projecting portion is the arc shape, the outer contour of the arc shape reduces the friction with the end of the sheath and the sheath wall, thereby further reducing the loading resistance.

Certainly, any one product for implementing this invention is unnecessary to achieve all the above advantages at the same time.

### BRIEF DESCRIPTION OF THE DRAWINGS

Fig. 1 is a structural schematic diagram of a stent assembly according to Embodiment 1 of this invention;
Fig. 2 is a structural schematic diagram of an inner layer stent according to Embodiment 1 of this invention;
Fig. 3 is a structural schematic diagram of an outer layer stent according to Embodiment 1 of this invention;
Fig. 4 is a structural schematic diagram of a sheath inserting end set according to Embodiment 1 of this invention;
Fig. 5 is a partial structural schematic diagram of a first sheath inserting end according to Embodiment 1 of this invention;
Fig. 6 is a partial cross-section diagram of a valve prosthesis according to Embodiment 1 of this invention;
Fig. 7 is a partial cross-section diagram of inserting the valve prosthesis into a sheath according to Embodiment 1 of this invention;
Fig. 8 is a cross-section structural schematic diagram of a sheath inserting end set according to Embodiment 1 of this invention;
Fig. 9 is a cross-section structural schematic diagram of a sheath inserting end set according to Embodiment 2 of this invention;
Fig. 10 is a cross-section structural schematic diagram of a sheath inserting end set according to Embodiment 3 of this invention;
Fig. 11 is a structural schematic diagram of a valve stent and a sheath inserting end in the prior art according to this invention;
Fig. 12 is a partial structural schematic diagram of inserting the valve prosthesis into the sheath in the prior art according to this invention.

References for numerals: stent assembly-100; inner layer stent-110; outer layer stent-120; inflow segment-111; outflow segment-113; transition segment-112; first segment-121; second segment-122; third segment-123; sheath inserting end set-101; first sheath inserting end-114; second sheath inserting end-124; projecting portion-200; sheath-410; first end surface-201; second end surface-202; end surface of first sheath inserting end-1141; end surface of second sheath inserting end-1241.

### DESCRIPTION OF THE PREFERRED EMBODIMENTS

This invention provides a valve stent and a prosthetic heart valve including the valve stent.

The prosthetic heart valve of this invention may be used to be implanted into a left ventricle inflow tract to replace a native mitral valve, and may also be used as a tricuspid valve to be implanted into a right ventricle inflow tract.

The structure of the prosthetic heart valve of this invention will be described below with the mitral valve as an example, wherein the heart valve consists of a stent assembly 100, a valve and a skirt.

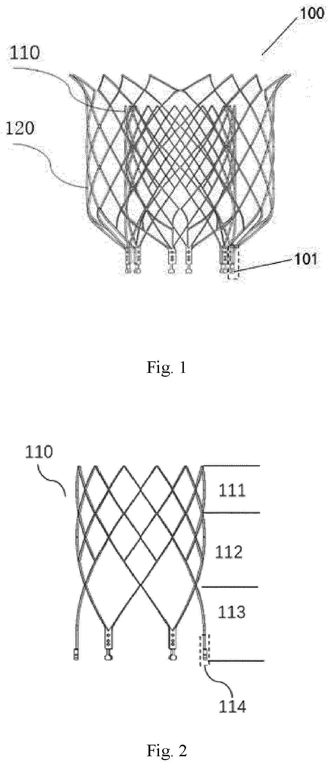

As shown in Fig. 1, the stent assembly 100 consists of an inner layer stent 110 and an outer layer stent 120, and the inner stent 110 and the outer layer stent 120 are of an internal-external nested structure, wherein the inner layer stent 110 is sleeved inside and the outer layer stent 120 is sleeved outside. The inner layer stent 110 is connected to the outer layer stent 120 by way of riveting, welding, snapping, stitching, and covering with skirts, and so on.

As shown in Fig. 2, the inner layer stent 110 includes an inflow segment 111, an outflow segment 113, and a transition segment 112 located between the inflow segment and the outflow segment; optionally, the inner layer stent 110 further includes a hanging tab. The outflow segment 113 is located downstream of the inflow tract according to a direction of the blood flow, the hanging tab is connected to an end portion of the stent inflow segment 111 and/or an end portion of the stent outflow segment 113, and the hanging tab is used to be connected to a delivery system, so as to ensure that the relative position of the valve prosthesis and the delivery system remains unchanged when the valve is loaded into the delivery system, the valve is released and removed from the delivery system and the valve is transported in the delivery system in vitro.

A cross-section of the inner layer stent 110 may be of a circle, a D-shape, a flower shape or other irregular shapes. The material used for preparing the inner layer stent 110 may be selected from such as nitinol, titanium alloy, cobalt chromium alloy, MP35n, 316 stainless steel, L605, Phynox/Elgiloy or platinum chromium, or may be prepared by other bio-compatible metals known by those skilled in the art. Optionally, the material used for preparing the inner layer stent may also include an elastic deformable material or a plastic deformable material such as a balloon-expandable material, or may include a shape memory alloy that may respond to temperature changes to convert between a compressed state and an expanded state. Preferably, the inner layer stent is prepared by cutting a nickel-titanium alloy pipe, and an outer diameter of the pipe is 4mm to 13mm, wherein the shaped diameter size should be selected according to actual needs. The inner layer stent 110 may be of a mesh structure constituted by a plurality of rows of units, and the constituent unit is a mesh unit that can be formed into an enclosed shape such as a triangle, a diamond, a pentagon, and a droplet shape, preferably the diamond structure.

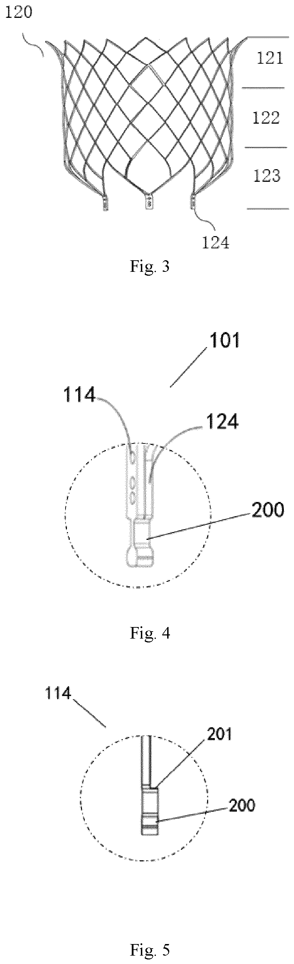

As shown in Fig. 3, the outer layer stent 120 includes a first segment 121, a second segment 122 and a third segment 123. Optionally, the outer layer stent 120 further includes a fixing tab, and the fixing tab is disposed on any end portion or two end portions of the outer layer stent 120. The fixing tab is used to be connected to the delivery system, so as to ensure that the relative position of the valve prosthesis and the delivery system remains unchanged when the valve is loaded into the delivery system, the valve is released and removed from the delivery system and the valve is transported in the delivery system in vitro.

Any one of or both the fixing tab and the hanging tab may be selected according to the releasing needs in order to be connected to the delivery device when the entire valve stent is loaded, and may be located on any one of the end portions of the stent and not limited to be located on the outflow segment.

A cross-section of the outer layer stent 120 may be of a circle, a D-shape, a flower shape or other irregular shapes. The outer layer stent 120 may adopt such as nitinol, titanium alloy, cobalt chromium alloy, MP35n, 316 stainless steel, L605, Phynox/Elgiloy or platinum chromium, or may be prepared by other bio-compatible metals known by those skilled in the art. Optionally, the material used for preparing the outer layer stent may also include an elastic deformable material or a plastic deformable material such as a balloon-expandable material, or may be a shape memory alloy that may respond to temperature changes to convert between a compressed state and an expanded state. The outer layer stent 120 may be of a mesh structure constituted by a plurality of rows of units, and the constituent unit is a mesh unit that can be formed into an enclosed shape such as a triangle, a diamond, a pentagon, and a droplet shape.

The valve includes at least two prosthetic valve leaflets, which are prepared by animal pericardium or other bio-compatible polymeric materials, wherein one end of the valve leaflet is directly or indirectly connected to the inner layer stent 110 stably, and the other end of the valve leaflet is a free end. The number of the valve leaflet may be the same as or different from the number of the native valve leaflet. When the heart valve is in a working state, the native valve leaflets are replaced by the prosthetic valve leaflets to realize functions of opening and closing a blood channel.

The overall inner surface or the overall outer surface or the two surfaces covered with skirts of the stent assembly 100 realizes sealing functions, so as to ensure that a single channel of the blood has a direction from an inflow tract end of the prosthesis valve leaflet to the outflow tract end of the prosthesis valve leaflet. The skirt is prepared by pericardium or other bio-compatible polymeric materials (e.g., PET (polyethylene terephthalate), PTFE (poly tetra fluoroethylene), etc.).

In this invention, the anchoring form for the valve prosthesis is not limited, and a flange may be disposed on the stent assembly 100, wherein an anchoring form of Oversize is used; or anchoring structures such as stabs and claws may also be disposed on the stent assembly 100 for grabbing the primary tissue; or the way in which the tether is anchored to the ventricle wall is also used for fixing.

In this invention, only the mitral valve prosthesis is used as an example, and this invention is not limited to an aortic valve prosthesis, a pulmonary valve prosthesis and a tricuspid valve prosthesis and the like with the similar structure.

As shown in Figs. 11 and 12, in the prior art, there is a problem of forming a gradient of sheath inserting end surfaces between the inner and outer stents when the length of the sheath inserting end of the inner layer stent 100 is greater than that of the outer layer stent 120. When being loaded, the end surface of the sheath inserting end of the outer layer stent 120 may touch the end surface of the sheath, and the two end surfaces may abut against each other, so that the entire valve may be affected in entering the sheath, the loading resistance is large and the sheath inserting end of the outer layer stent 120 may easily damage the sheath. During the implanting process, a retrievable resistance is large if the retrieve and deployment are required, which will also affect the smooth progress of the retrieve.

In addition, when there is a gradient difference between the sheath inserting ends of the inner and outer layer stents, there exists the risk of the sheath entering the interlayer of the two layer stents, even the risk of the sheath entering the interlayer of the inner and outer stents.

As such, this invention provides a valve stent and a valve prosthesis, which may solve the above problems.

In the description of this invention, it should be noted that since one end or two ends of the valve prosthesis are required to be loaded into the delivery system during the loading process, the end entering the delivery system first is the "sheath inserting end" . The term "sheath inserting end" should be understood broadly, which is an end portion structure disposed at the atrium end or the ventricle end of the valve prosthesis, such as the above "hanging tab" and "fixing tab" .

In the description of this invention, it should be noted that the "atrium end" refers to an end portion located in the atrium, and the "ventricle end" refers to an end portion located in the ventricle. As mentioned here, "heart valve" , "valve prosthesis" and "prosthetic heart valve " have the same meaning.

In the description of this invention, it should be noted that the " outer side " refers to a direction facing externally along a radial direction from a center of the valve prosthesis. As mentioned here, the "length" refers to a size along an axial direction, and the "width" refers to a size along the radial direction.

In the description of this invention, it should be noted that orientations or position relationships indicated by terms " center" , "upper" , "lower" , "left" , "right" , "vertical" , "horizontal" , " inside " , " outside " and the like are orientations or position relationships shown in the drawings, and these terms are merely for facilitating description of this invention and simplifying the description, but not for indicating or implying that the mentioned device or elements must have a specific orientation and must be established and operated in a specific orientation, and thus, these terms cannot be understood as a limitation to this invention. Moreover, terms like "first", "second", "third" etc. are only used for description, not be considered as a designation or designation of relative importance.

In the description of this invention, it should be noted that, unless otherwise clearly specified and limited, meanings of terms "install", "connected with", and "connected to" should be understood in a wide sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in this invention may be understood based on specific situations.

As used in the specification, singular forms "a/an," "one," and "the/that" include plural objects, unless otherwise explicitly stated. As used in the specification, the term "or" is usually used to include the meaning of "and/or" , unless otherwise expressly stated.

The following further describes this invention in combination with specific embodiments.

### Embodiment 1

This invention embodiment provides a valve stent including a stent assembly 100, with reference to Figs. 1 to 8, wherein the stent assembly 100 includes two stents: an inner layer stent 110 and an outer layer stent 120, and the inner stent 110 and the outer layer stent 120 are of an internal-external nested structure, wherein the inner layer stent 110 is sleeved inside and the outer layer stent 120 is sleeved outside.

The stent assembly 100 is configured to be provided with at least one sheath inserting end set 101, and the at least one sheath inserting end set 101 includes a first sheath inserting end 114 and a second sheath inserting end 124, wherein the first sheath inserting end 114 is located at the inner layer stent 110, the second sheath inserting end 124 is located at the outer layer stent 120, the first sheath inserting end 114 and the second sheath inserting end 124 extend in the same direction, and the second sheath inserting end 124 is located on an outer side of the first sheath inserting end 114.

In the at least one sheath inserting end set 101, a length of the first sheath inserting end 114 is greater than a length of the second sheath inserting end 124, wherein the at least one sheath inserting end set 101 is configured with at least one projecting portion 200 for preventing the loading of the valve prosthesis from being interfered by the sheath inserting end set 101.

Since the second sheath inserting end 124 is located on the outer side of the first sheath inserting end 114, when the length of the first sheath inserting end 114 is greater than the length of the second sheath inserting end 124, i.e., when there is a gradient different between the two sheath inserting ends, the end surface of the second sheath inserting end 124 on the outer side may touch the end surface of the sheath 410 in the sheath inserting process, and the two end surfaces may abut against each other, so that the loading resistance is larger, thereby inserting the entire valve prosthesis into the sheath 410 is affected.

The provision of the projection portion 200 may avoid the above conditions, which includes preventing the sheath 410 from entering a gap between the first sheath inserting end 114 and the second sheath inserting end 124, or preventing the end surface of the sheath 410 from abutting against the end surface of the second sheath inserting end 124 and hence preventing inserting the valve prosthesis into the sheath from being affected.

In this invention embodiment, the ventricle end of the valve prosthesis is first inserted into the sheath, i.e., the ventricle end being the sheath inserting end. The first sheath inserting end 114 is the hanging tab disposed at the ventricle end of the inner layer stent 110, the first sheath inserting end 114 is first inserted into the sheath, and the hanging tab first inserted into the sheath is fixed with the transporting system, which is reliable and stable; the second sheath inserting end 124 may not have any functions, e.g., being an end point of the outer layer stent 120. Naturally, in other embodiments, the first sheath inserting end 114 or the second sheath inserting end 124 may also be a connecting rod having a pre-set function, and specific structures and functions of the first sheath inserting end 114 and the second sheath inserting end 124 are not used to limit the protection scope of this invention.

Further, with reference to Fig. 1, in order to cause the valve prosthesis to be stably fixed in the transporting sheath and to enable smooth releasing of the valve prosthesis, the ventricle end of the stent assembly 100 is provided with a plurality of sheath inserting end sets 101, and the plurality of sheath inserting end sets 101 are arranged uniformly and circumferentially; in each of the sheath inserting end sets 101, when the length of the first sheath inserting end 114 is greater than the length of the second sheath inserting end 124, each of the sheath inserting end sets 101 is configured with the projecting portion 200. In this way, the condition that the sheath enters the interlayer of any one of the sheath inserting end sets 101 and any one of the second sheath inserting ends 124 abuts against the end surface of the sheath may be prevented during the sheath inserting process, so that the valve prosthesis may be inserted into the sheath smoothly. Naturally, in other embodiments, the plurality of sheath inserting end sets 101 may be disposed at the atrium end of the stent assembly 100 simultaneously, or both the atrium end and the ventricle end of the stent assembly 100 are provided with the plurality of sheath inserting end sets 101. The position and number where the sheath inserting end set 101 is disposed should be selected according to actual releasing ways and the structure of the delivery system, which will not repeated here.

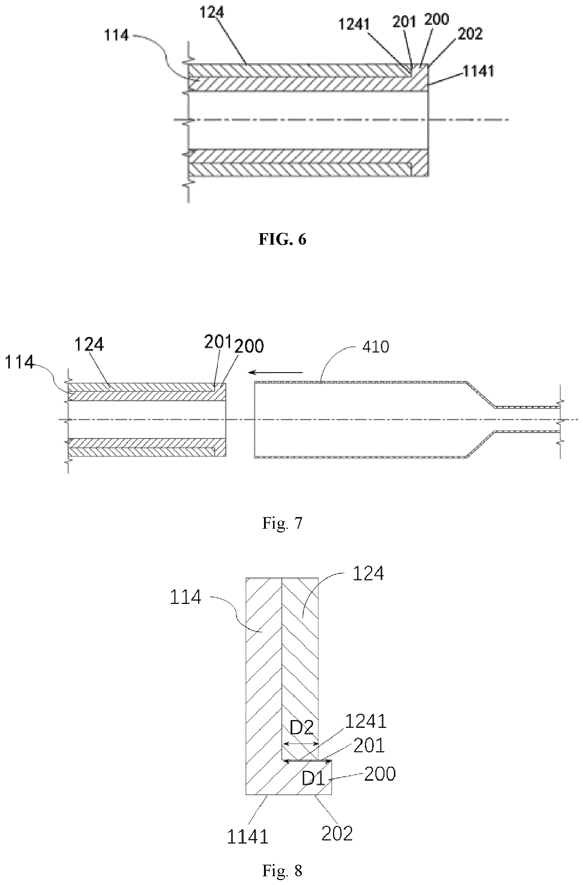

With reference to Figs. 6 and 8, the end surface of the first sheath inserting end 1141 is located at a free end of the first sheath inserting end 114, and the end surface of the second sheath inserting end 1241 is located at a free end of the second sheath inserting end 124.

In this invention embodiment, the projecting portion 200 is disposed at the first sheath inserting end 114, the projecting portion 200 is provided with a first end surface 201, and the first end surface 201 is located on a side close to the end surface of the second sheath inserting end 1241, wherein a width D1 of the first end surface 201 is not smaller than a width D2 of the second sheath inserting end 124.

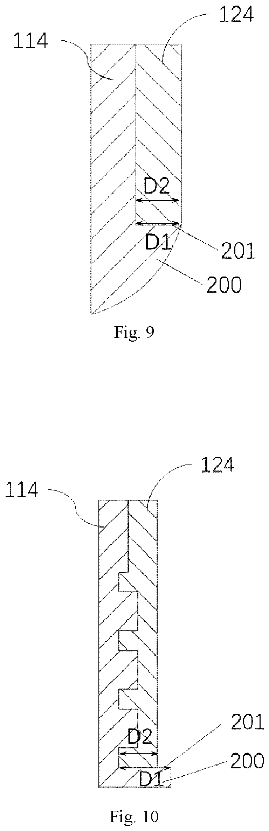

The width D1 of the first end surface 201 may be equal to the width D2 of the second sheath inserting end 124, as shown in Figs. 6 and 7. In some embodiments, the width D1 of the first end surface may also be greater than the width D2 of the second sheath inserting end 124, as shown in Figs. 8 and 10. When there is a gradient difference between the first sheath inserting end 114 and the second sheath inserting end 124 and the two are pressed and held into the sheath, the sheath easily enters the gap between the first sheath inserting end 114 and the second sheath inserting end 124, and the projecting portion 200 disposed at the first sheath inserting end 114 and extending outward may reduce the risk of the sheath entering the gap between the first sheath inserting end 114 and the second sheath inserting end 124.

In this invention embodiment, with reference to Figs. 6 and 8, the projecting portion 200 is further provided with a second end surface 202, and the second end surface 202 is located on a side deviated from the first end surface 201, wherein the second end surface 202 of the projecting portion 200 is flush with the end surface of the first sheath inserting end 1141. Then, the structure of the projecting portion 200 removes the gradient difference between the two sheath inserting ends, as shown in Fig. 7, and in the sheath inserting process, the end surface of the second sheath inserting end 124 may not touch the end surface of the sheath; therefore, the end surface of the second sheath inserting end 124 may be effectively prevented from abutting against the end surface of the sheath, so that the loading resistance is reduced and the second sheath inserting end 124 may not damage the sheath. Naturally, in other embodiments, the projecting portion 200 may also extend towards the ventricle end to exceed the end surface of the first sheath inserting end 1141, which similarly may solve the problem that the second sheath inserting end 124 abuts against the sheath 410 in the sheath inserting process, which is not limited here.

Further, in this invention embodiment, the first end surface 201 of the projecting portion 200 abuts against the end surface of the second sheath inserting end 1241. Since being subjected to the abutting action from the projecting portion 200, in the sheath inserting process, the sheath 410 may not enter the interlayer of the stents from the second sheath inserting end 124.

With continuous reference to Figs. 4, 6 and 7, in this invention embodiment, the first end surface 201 of the projecting portion 200 is matched with the end surface of the second sheath inserting end 1241. Being matched with refers to the two have the same size and to that the two end surfaces form a concave-convex match; therefore, when the first sheath inserting end 114 and the second sheath inserting end 124 abut against each other, each of the sheath inserting end sets 101 serves as a whole, which reduces the risk of the sheath 410 entering the interlayer, thereby making the loading and releasing process of the valve prosthesis more smoothly.

In this invention embodiment, the projecting portion 200 is formed integrally at the first sheath inserting end 114, which has the advantages of simple, stable and reliable molding process. Naturally, in other alternative embodiments, the projecting portion 200 may be independently molded and then fixedly connected to the first sheath inserting end 114; or the projecting portion 200 may also movably be connected on the first sheath inserting end 114, e.g., by way of movable connection. Or, the projecting portion 200 may also be configured as a removable structure covering on the free end of the sheath inserting end set 101, and then the projecting portion 200 should be made of a biodegradable material, such as polylactic acid.

In this invention embodiment, the projecting portion 200 may also have a certain function, such as being used as the hanging tab or the fixing tab.

### Embodiment 2

This invention embodiment provides a valve stent, which is an improvement made based on Embodiment 1, wherein an outer contour of the projecting portion 200 is of an arc shape.

As shown in Fig. 9, since the first end surface 201 of the projecting portion 200 abuts against the end surface of the sheath inserting end 1241, the arc-shaped contour may reduce the friction with the sheath and an inner wall of the sheath, thereby further reducing the loading resistance.

### Embodiment 3

This invention embodiment provides a valve stent, which is an improvement made based on Embodiment 1 or 2, wherein an surface where the first sheath inserting end 114 contact axially the second sheath inserting end 124 is configured to be in the concave-convex matched connection.

With reference to Fig. 10, a plurality of second protrusions are protruded outward from an axial side of the second sheath inserting end 124, and a second concave portion is formed between the adjacent two second protrusions; correspondingly, an axial side of the first sheath inserting end 114 forms a plurality of first concave portions, and a first protrusion is formed between the adjacent two first concave portions 116. After the inner layer stent 110 is connected to the outer layer stent 120, the first protrusion is located in one of the corresponding second concave portions respectively, and the second protrusion is located in one of the corresponding first concave portions respectively, thereby enabling the concave-convex matched connection to be formed between the first sheath inserting end 114 and the second sheath inserting end 124. The protrusions are in matched connection with the concave portions one by one, and the number and arrangement position of the protrusions and the concave portions may be selected according to actual connection needs and are not used to limit the protection scope of this invention, which will be not repeated here.

The concave-convex matched connection between the first sheath inserting end 114 and the second sheath inserting end 124 enable each of the sheath inserting end sets 101 to be pre-molded, and may replace connection ways such as welding, thereby having advantages of firm connection and being easy to implement.

The above disclosure is only the preferred embodiment of this invention. The preferred embodiments do not describe all the details, and are not intended to limit the invention only to be the specific embodiments. It is obvious that various modifications and changes can be made to the content of the specification. This invention selects and specifically describe the embodiments with the purpose of better explain the principle and practical use of this invention, such that a person skilled in the art can well utilize this invention. This invention is merely limited by the appended claims and the scope and equivalents thereof.

## Claims

1. A valve stent, comprising a stent assembly, wherein the stent assembly comprises at least two stents: an inner layer stent and an outer layer stent, and the inner layer stent and the outer layer stent are of an internal-external nested structure; the inner layer stent is sleeved inside, and the stent assembly is configured to have at least one sheath inserting end set;
the sheath inserting end set comprises a first sheath inserting end and a second sheath inserting end, the first sheath inserting end is located at the inner layer stent, and the second sheath inserting end is located at the outer layer stent; the first sheath inserting end and the second sheath inserting end extend in a same direction, and the second sheath inserting end is located on an outer side of the first sheath inserting end;
in the at least one sheath inserting end set, when a length of the first sheath inserting end is greater than a length of the second sheath inserting end, the sheath inserting end set is configured with a projecting portion for preventing the loading of a valve prosthesis from being interfered by the sheath inserting end set.

2. The valve stent according to claim 1, wherein the stent assembly comprises a plurality of the sheath inserting end sets, and each of the sheath inserting end sets is located at an atrium end or a ventricle end; in each of the sheath inserting end sets, when the length of the first sheath inserting end is greater than the length of the second sheath inserting end, each of the sheath inserting end sets is configured with the projecting portion.

3. The valve stent according to claim 2, wherein the projecting portion is arranged at the first sheath inserting end, the projecting portion is provided with a first end surface, the first end surface is located on a side close to an end surface of the second sheath inserting end, and a width of the first end surface is not smaller than a width of the second sheath inserting end.

4. The valve stent according to claim 3, wherein the projecting portion is further provided with a second end surface, the second end surface is located on a side deviated from the first end surface, and the second end surface is flush with the end surface of the first sheath inserting end.

5. The valve stent according to claim 3, wherein the first end surface of the projecting portion abuts against the end surface of the second sheath inserting end.

6. The valve stent according to claim 5, wherein a shape of the first end surface is matched with a shape of the end surface of the second sheath inserting end.

7. The valve stent according to any one of claims 1-6, wherein an outer contour of the projecting portion is an arc shape.

8. The valve stent according to any one of claims 3-6, wherein the projecting portion is formed integrally at the first sheath inserting end.

9. The valve stent according to claim 1, wherein an axial contact surface between the first sheath inserting end and the second sheath inserting end is configured to be a concave-convex matched connection.

10. The valve stent according to claim 1, wherein the first sheath inserting end is a hanging tab connected to a delivery system.

11. A prosthetic heart valve comprising the valve stent according to any one of claims 1 to 10.
